# EUROPEAN PATENT SPECIFICATION

(11) **EP 1 591 079 B1**
(45) Date of publication and mention of the grant of the patent: **20.02.2008**
(21) Application number: 05252657.1
(22) Date of filing: 28.04.2005
(51) Int. Cl.: A61F 2/82

(54) **Locking stent having multiple locking points**
Stent mit Mehrpunktverschlussmechanismus
Stent à verrouillage à plusieurs points de verrouillage

(30) Priority: 29.04.2004 US 834687
(43) Date of publication of application: 02.11.2005
(73) Proprietor: Cordis Corporation, Miami Lakes, Florida 33014 (US)
(72) Inventor: Fleming, James A., III, Bethlehem, PA 18020 (US); Vipul Bhupendra, Dave, Hillsborough, NJ 08844 (US)
(74) Representative: Mercer, Christopher Paul

(56) References cited:
- EP-A- 1 266 638
- EP-A- 1 452 152
- WO-A-99/01087
- WO-A-02/062268
- WO-A-02/065949

## Description

### FIELD OF THE INVENTION

The present invention relates, in general, to intralumenal medical devices, and, more particularly, two a new and useful stent having interlocking elements with multiple locking points for stenting a vessel.

### Background Art

A stent is commonly used as a tubular structure left inside the lumen of a duct to relieve an obstruction. Commonly, stents are inserted into the lumen in a non-expanded form and are then expanded autonomously (or with the aid of a second device) *in situ*. When used in coronary artery procedures for relieving stenosis, stents are placed percutaneously through the femoral artery. In this type of procedure, stents are delivered on a catheter and are either self-expanding or, in the majority of cases, expanded by a balloon. Self-expanding stents do not need a balloon to be deployed. Rather the stents are constructed using metals with spring-like or superelastic properties (i.e., Nitinol), which inherently exhibit constant radial support. Self-expanding stents are also often used in vessels close to the skin (i.e., carotid arteries) or vessels that can experience a lot of movement (i.e., popliteal artery). Due to a natural elastic recoil, self-expanding stents withstand pressure or shifting and maintain their shape.

As mentioned above, the typical method of expansion for balloon expanded stents occurs through the use of a catheter mounted angioplasty balloon, which is inflated within the stenosed vessel or body passageway, in order to shear and disrupt the obstructions associated with the wall components of the vessel and to obtain an enlarged lumen.

Balloon-expandable stents involve crimping the device onto an angioplasty balloon. The stent takes shape as the balloon is inflated and remains in place when the balloon and delivery system are deflated and removed.

In addition, balloon-expandable stents are available either pre-mounted or unmounted. A pre-mounted system has the stent already crimped on a balloon, while an unmounted system gives the physician the option as to what combination of devices (catheters and stents) to use. Accordingly, for these types of procedures, the stent is first introduced into the blood vessel on a balloon catheter. Then, the balloon is inflated causing the stent to expand and press against the vessel wall. After expanding the stent, the balloon is deflated and withdrawn from the vessel together with the catheter. Once the balloon is withdrawn, the stent stays in place permanently, holding the vessel open and improving the flow of blood.

In the absence of a stent, restenosis may occur as a result of elastic recoil of the stenotic lesion. Although a number of stent designs have been reported, these designs have suffered from a number of limitations. Some of these limitations include design limitations resulting in low radial strength, decrease in the length of the stent upon deployment, i.e. foreshortening, and high degree of axial compression experienced by the stent.

The radial strength issue was addressed in WO 99/01087, but it still foreshortens. Its features form the basis for the preamble of claim 1 appended hereto. The radial strength issue was also addressed in EP 1 452 152, published on 1st September 2004, ie. after the priority date of the present invention which constitutes prior art under Article 54(3) EPC.

### Brief Summary of the Invention

The present invention relates to an apparatus for stenting a vessel in conjunction with a particular new and useful stent having a lattice of interconnecting elements defining a substantially cylindrical configuration. The lattice has a first open end and a second open end wherein the lattice is movable between a closed configuration and a final open configuration, as defined in claim 1.

Each hoop preferably comprises a plurality of loops. And, more preferably, each hoop further comprises a plurality of struts connected to the loops.

Preferably, at least one loop of one hoop comprises a male end and at least one loop of another hoop comprises a female end. The male end is separated from the female end when the lattice is in the closed configuration. The male end is connectably mated to the female end when the lattice is moved to the final open configuration thereby locking the stent lattice in the final open configuration.

Preferably, the male end of at least one loop of one hoop and the female end of at least one loop of another hoop form a locked joint when the lattice is moved into the final open configuration thereby locking the stent in the open configuration.

Preferably, the lattice further comprises at least one flexible link or a plurality of flexible links connected between adjacent hoops. The flexible links may comprise various shapes such as a sinusoidal shaped, straight or linear shape, or a substantially S-shaped or Z-shaped pattern. At least one flexible link is connected between loops of adjacent hoops of the lattice.

Preferably, the plurality of struts and the loops define at least one pre-configured cell. Preferably, the lattice comprises a plurality of pre-configured cells defined by the plurality of struts and the loops of the lattice.

Preferably, the plurality of struts and the loops also define at least one partial cell. In a preferred embodiment in accordance with the present invention, the plurality of struts and the loops define a plurality of partial cells. A partial cell is defined by the plurality of struts and the loops when the lattice is in the closed configuration.

Preferably, the plurality of struts and the loops define at least one formed cell. In a preferred embodiment in accordance with the present invention, the plurality of struts and the loops of the stent lattice define a plurality of formed cells. A formed cell is defined by the plurality of struts and the loops when the lattice is moved into the open configuration (locked configuration).

Preferably the male end of the at least one loop of one hoop has a substantially convex configuration. The female end of at least one loop of another hoop has a substantially concave configuration. In accordance with the present invention, alternative forms, shapes or configurations for the male end and female end respectively are also contemplated herein.

In accordance with one embodiment of the present invention, each pre-configured cell has a substantially diamond shape. Other shapes for the pre-configured cell are also contemplated by the present invention, and thus, the pre-configured cell may take the form of any desired shape.

The stent of the present invention is directed toward both a balloon actuated stent and a self-expanding stent. The stent is made of any suitable material. In one embodiment, the stent is made of an alloy such as stainless steel. In another preferred embodiment, the stent is made of a nickel titanium (Nitinol) alloy. Moreover, this material or any other super-elastic alloy is suitable for the stent according to the present invention. In these self-expanding stent embodiments, the stent is a crush recoverable stent.

The lattice comprises a plurality of adjacent hoops, wherein at least two hoops are movable to one or more discrete locked positions as the open configuration and at least one hoop interlocks with another hoop at the one or more discrete locked positions.

Preferably at least one hoop interlocks with another hoop when the lattice is in a final open configuration. However, at least one hoop interlocks with another hoop at a plurality of points while the lattice is moved from the closed configuration towards the final open configuration.

In some embodiments according to the present invention the lattice is in a locked position when the stent is in a closed configuration, i.e. on a stent delivery device or catheter prior to deployment. Alternatively, in other embodiments according to the present invention, the lattice is in an unlocked position when the stent is in a closed configuration, i.e. on a stent delivery device or catheter prior to deployment.

As outlined above, each hoop may comprises a plurality of loops. And, each hoop may further comprise a plurality of struts connected to the loops. Furthermore, at least one strut of one hoop may interlock with a strut of an adjacent hoop such that the at least one strut of one hoop interlocking with the strut of an adjacent hoop define interlocking adjacent struts. The interlocking adjacent struts interlock with each other at a plurality of points.

The stent according to the present invention preferably comprises interlocking adjacent struts wherein each of these interlocking adjacent struts comprise a plurality of teeth mateably connectable and interlockingly movable with each other as the lattice is moved from the closed configuration to the open configuration.

In some embodiments according to the present invention the lattice further comprises a drug coating or a drug and polymer coating combination. In some embodiments according to the present invention, the drug is rapamycin. In other embodiments according to the present invention, the drug is paclitaxel. The drug can be any desired therapeutic agent such as any type of chemical compound, biological molecule, nucleic acids such as DNA and RNA, peptide, protein or combinations thereof.

Moreover, the stent according to the present invention may be made of a polymer, and in certain embodiments, the stent is made of a bioabsorbable or biodegradable polymer. The polymer can be either a bulk erodible or surface erodible polymer. In some embodiments where the stent is made of a biodegradable polymer, the stent further comprises a drug or any desired therapeutic agent such as those mentioned above and detailed later in this disclosure. In some of these embodiments, the drug is rapamycin. In other of these embodiments the drug is paclitaxel. Additionally, in some embodiments, the stent further comprises a radiopaque material.

### BRIEF DESCRIPTION OF THE DRAWINGS

The novel features of the invention are set forth with particularity in the appended claims. The invention itself, however, both as to organization and methods of operation, together with further objects and advantages thereof, may be best understood by reference to the following description, taken in conjunction with the accompanying drawings in which:
Fig. 1 is a perspective view of a of a stent in a closed-configuration;
Fig. 2 is a partial side plan view of the stent of Fig. 1A in the closed configuration;
Fig. 3 is a perspective view of the stent of Fig. 1 in an open configuration;
Fig. 4 is a partial side view of the stent of Fig. 1 in the open configuration;
Fig. 5 is a partial side view of an embodiment of a stent having multiple locking points in a closed configuration in accordance with the present invention;
Figs. 6A - 6E depict partial side views of the stent of Fig. 5 in discrete locked positions during various stages of moving the stent from the closed configuration to an open configuration in accordance with the present invention; and
Fig. 7 is a partial side view of the stent of Fig. 5 in this open configuration in accordance with the present invention.

### DETAILED DESCRIPTION OF THE INVENTION

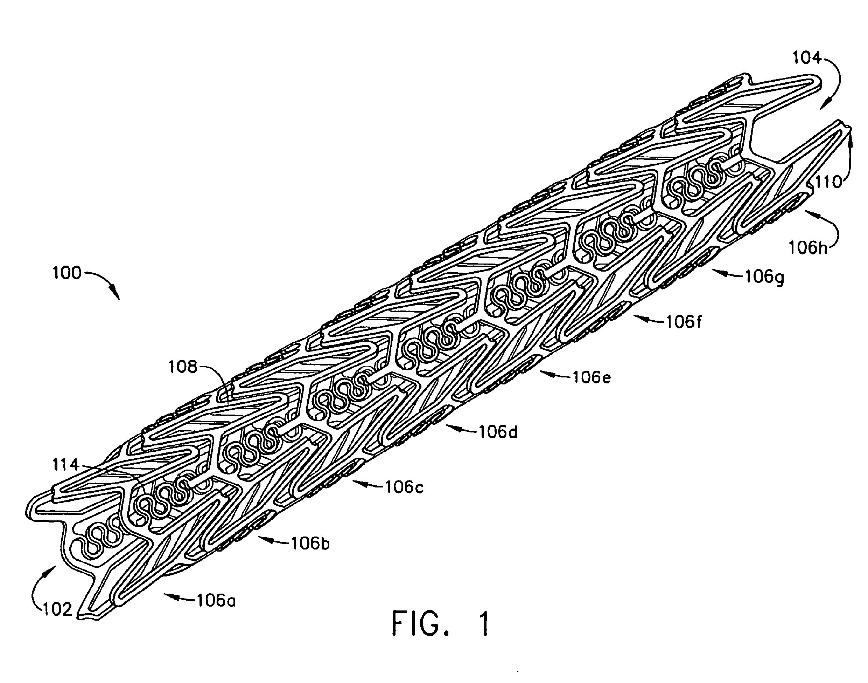

In Figs. 1-4, a stent 100 that is an expandable prosthesis for a body passageway is illustrated. It should be understood that the terms "stent" and "prosthesis" are interchangeably used to some extent in describing the present invention, insofar as the method, apparatus, and structures of the present invention may be utilized not only in connection with an expandable intraluminal vascular graft for expanding partially occluded segments of a blood vessel, duct or body passageways, such as within an organ, but may so be utilized for many other purposes as an expandable prosthesis for many other types of body passageways. For example, expandable prostheses may also be used for such purposes as: (1) supportive graft placement within blocked arteries opened by transluminal recanalization, but which are likely to collapse in the absence of internal support; (2) similar use following catheter passage through mediastinal and other veins occluded by inoperable cancers; (3) reinforcement of catheter created intrahepatic communications between portal and hepatic veins in patients suffering from portal hypertension; (4) supportive graft placement of narrowing of the esophagus, the intestine, the ureters, the uretha, etc.; (5) intraluminally bypassing a defect such as an aneurysm or blockage within a vessel or organ; and (6) supportive graft reinforcement of reopened and previously obstructed bile ducts. Accordingly, use of the term "prosthesis" encompasses the foregoing usages within various types of body passageways, and the use of the term "intraluminal graft" encompasses use for expanding the lumen of a body passageway. Further in this regard, the term "body passageway" encompasses any lumen or duct within the human body, such as those previously described, as well as any vein, artery, or blood vessel within the human vascular system.

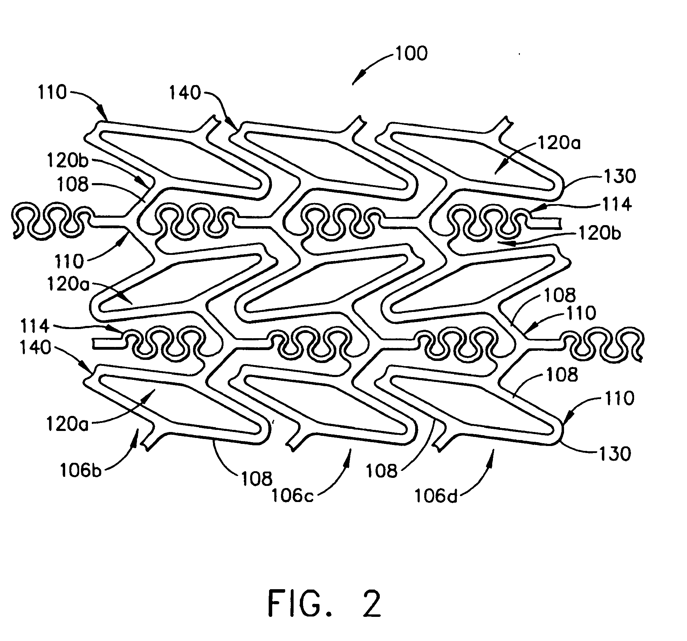

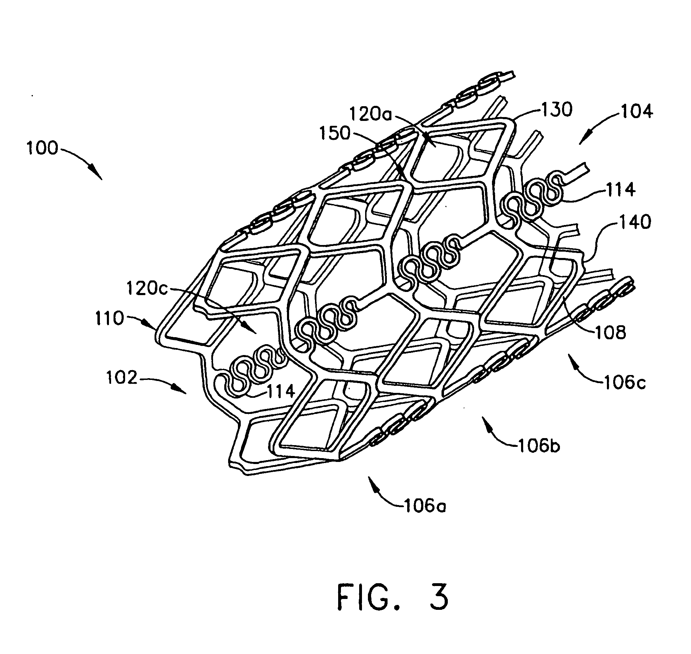

The stent 100 is an expandable lattice structure made of any suitable material which is compatible with the human body and the bodily fluids (not shown) with which the stent 100 may come into contact. The lattice structure is an arrangement of interconnecting elements made of a material which has the requisite strength and elasticity characteristics to permit the tubular shaped stent 100 to be moved or expanded from a closed (crimped) position or configuration shown in Figs. 1 and 2 to an expanded or open position or configuration shown in Figs. 2 and 3. Some examples of materials that are used for the fabrication of the stent 100 include silver, tantalum, stainless steel, gold, titanium or any type of plastic material having the requisite characteristics previously described. Based on the interlocking design of the stent 100 (greater detail provided later in this disclosure), when the stent 100 is deployed or expanded to its open position, even materials that tend to recoil to a smaller diameter or exhibit crushing or deformation-like properties are used for the stent 100 in accordance with the present invention. These are materials that are not used in traditional stent designs. Some examples of these non-traditional stent materials that are used for the stent 100 in accordance with the present invention include deformable plastics, plastics that exhibit crushing or recoil upon deployment of the stent or polymers such as biodegradable polymers. Thus, the stent 100 in accordance with the present invention is also made of these type of plastics or polymers to include biodegradable polymers. Additionally, the biodegradable polymers used as material for the stent 100 can be drug eluting polymers capable of eluting a therapeutic and/or pharmaceutical agent according to any desired release profile.

The stent may be fabricated from 316L stainless steel alloy. Preferably the stent 100 comprises a superelastic alloy such as nickel titanium (NiTi, e. g., Nitinol). More preferably, the stent 100 is formed from an alloy comprising from about 50.5 to 60.0% Ni by atomic weight and the remainder Ti. Even more preferably, the stent 100 is formed from an alloy comprising about 55% Ni and about 45% Ti. The stent 100 is preferably designed such that it is superelastic at body temperature, and preferably has an Af temperature in the range from about 24° C to about 37° C. The superelastic design of the stent 100 makes it crush recoverable and thus suitable as a stent or frame for any number of vascular devices for different applications.

The stent 100 comprises a tubular configuration formed by a lattice of interconnecting elements defining a substantially cylindrical configuration and having front and back open ends 102, 104 and defining a longitudinal axis extending therebetween. In its closed configuration, the stent 100 has a first diameter for insertion into a patient and navigation through the vessels and, in its open configuration, a second diameter, as shown in Fig. 3, for deployment into the target area of a vessel with the second diameter being greater than the first diameter. The stent 100 comprises a plurality of adjacent hoops 106a-106h extending between the front and back ends 102, 104. The stent 100 comprises any combination or number of hoops 106. The hoops 106a-106h include a plurality of longitudinally arranged struts 108 and a plurality of loops 110 connecting adjacent struts 108. Adjacent struts 108 or loops 110 are connected at opposite ends by flexible links 114 which can be any pattern such as sinusoidal shape, straight (linear) shape or a substantially S-shaped or Z-shaped pattern. The plurality of loops 110 have a substantially curved configuration.

The flexible links 114 serve as bridges, which connect adjacent hoops 106a-106h at the struts 108 or loops 110. Each flexible link comprises two ends wherein one end of each link 114 is attached to one strut 108 or one loop 110 on one hoop 106a and the other end of the link 114 is attached to one strut 108 or one loop 110 on an adjacent hoop 106b, etc.

The above-described geometry better distributes strain throughout the stent 100, prevents metal to metal contact where the stent 100 is bent, and minimizes the opening between the features of the stent 100; namely, struts 108, loops 110 and flexible links 114. The number of and nature of the design of the struts, loops and flexible links are important design factors when determining the working properties and fatigue life properties of the stent 100. It was previously thought that in order to improve the rigidity of the stent, struts should be large, and thus there should be fewer struts 108 per hoop 106a-106h. However, it is now known that stents 100 having smaller struts 108 and more struts 108 per hoop 106a-106h improve the construction of the stent 100 and provide greater rigidity. Preferably, each hoop 106a-106h has between twenty-four (24) to thirty-six (36) or more struts 108. It has been determined that a stent having a ratio of number of struts per hoop to strut length which is greater than four hundred has increased rigidity over prior art stents which typically have a ratio of under two hundred. The length of a strut is measured in its compressed state (closed configuration) parallel to the longitudinal axis of the stent 100 as illustrated in Fig. 1.

Fig. 3 illustrates the stent 100 in its open or expanded state. As may be seen from a comparison between the stent 100 configuration illustrated in Fig. 1 and the stent 100 configuration illustrated in Fig. 3, the geometry of the stent 100 changes quite significantly as it is deployed from its unexpanded state (closed or crimped configuration/position) to its expanded state (open or expanded configuration/position). As the stent 100 undergoes diametric change, the strut angle and strain levels in the loops 110 and flexible links 114 are affected. Preferably, all of the stent features will strain in a predictable manner so that the stent 100 is reliable and uniform in strength. In addition, it is preferable to minimize the maximum strain experienced by the struts 108, loops 110 and flexible links 114 since Nitinol properties are more generally limited by strain rather than by stress. The embodiment illustrated in Figs. 1-4 has a design to help minimize forces such as strain.

As best illustrated in Fig. 2, the stent 100, in the closed-configuration (crimped configuration wherein the stent 100 is crimped on the stent delivery device such as a catheter), has a plurality of pre-configured cells 120a. Each pre-configured cell 120a is defined by the struts 108 and loops 110 connected to each other respectively thereby defining an open area in the stent lattice 100. This open area is a space identified as the pre-configured cell 120a.

Each hoop 106a-106h has one or more (or a plurality of) pre-configured cells 120a. In one embodiment according to the present invention, the pre-configured cell 120a is a diamond-shaped area or space. However, it is contemplated in accordance with the present invention that the pre-configured cell 120a take the form of any desired alternative shape.

Additionally, the stent lattice 100 also includes at least one (or a plurality of) partial cells 120b. Each partial cell 120b is defined by struts 108 and one loop 110 of the respective hoops 106a-106h. In one embodiment according to the present invention, the partial cell 120b defines a semi-enclosed area or space having an open end in direct communication with a loop 110 from an adjacent hoop 106a-106h. In this embodiment according to the present invention, the flexible link 114 connects adjacent hoops, for example hoop 106b to hoop 106c, by having one end of flexible link 114 connected to an inner surface of loop 110 of a partial cell 120b of the hoop 106b and the opposite end of the flexible link 114 connected to loop 110 of the adjacent hoop 106c. Thus, the flexible link 114 extends from one end of the partial cell 120b, for instance, of hoop 106b and extends through the semi-enclosed area of the partial cell 120b and is connected to loop 110 of the adjacent hoop 106c. The flexible links 114 are connected between adjacent hoops 106a-106h by extension through the partial cells 120b. Additionally, the partial cell 120b is not only a semi-enclosed area or space defined by struts 108 and one loop 110 of each hoop 106, but the partial cell 120b may take the form of any desired semi-enclosed shape.

Each partial cell 120b of the stent lattice 100 exists while the stent 100 is in its crimped state or closed configuration, i.e. crimped to the delivery device such as a catheter.

Moreover, each pre-configured cell 120a may have one loop 110 terminating in a male end 130 and the other loop defining the pre-configured cell 120a terminating in a female and 140. Thus, the male end 130 of one loop 110 and the female end 140 of the other loop 110 of the pre-configured cell 120a are positioned opposite each other thereby defining opposite ends of the pre-configured cell 120a, for example opposite ends of the diamond-shaped area in this embodiment.

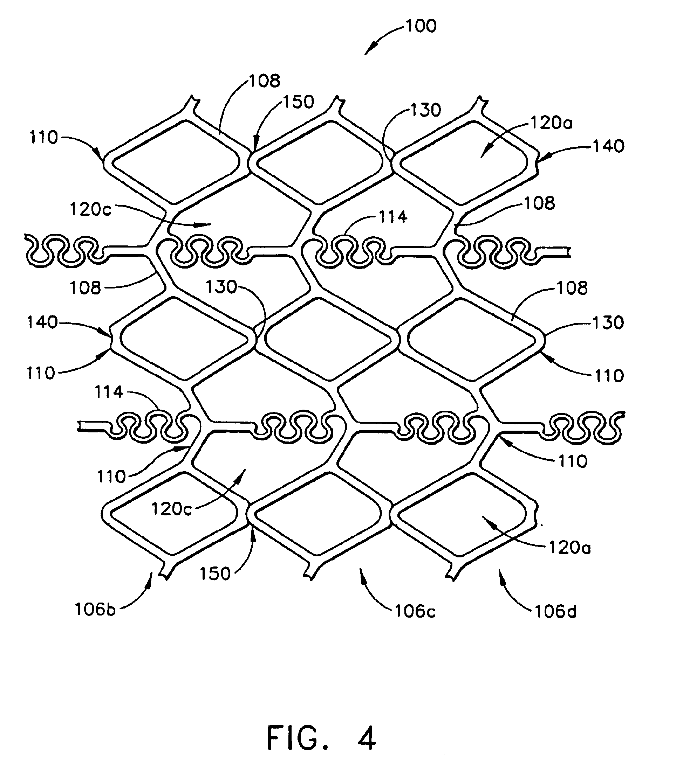

The male end 130 may have a substantially convex configuration and the female end 140 has a substantially concave configuration. In general, the female end 140 is designed such that it is shaped to receive and mateably connect with the male end 130. Accordingly, in this embodiment, the substantially concave surface of the female end 140 mateably connects with the substantially convex shape of the male end 130 when the stent lattice 100 is moved to the open configuration or state (deployed or expanded state) such as shown in Figs. 3 and 4.

As best illustrated in Fig. 4, when the stent lattice 100 is deployed or expanded to its open position or configuration, the male end 130 of the loop 110 of one hoop 106, for example 106b, mateably connects with the female end 140 of an opposite loop 110 of an adjacent hoop, for example 106c, thereby forming a locked joint 150. The male end 130 and the female end 140 may take the form of any desired shape or configuration that permits the male end 130 to mateably connect with the female end 140 in order to form the locked joint 150. For example, the male end 130 and the female end 140 may be shaped respectively in order to form portions of a dove-tail such that the locked joint 150 has or forms a dove-tail configuration. Other shapes for the male end 130 and female end 140 forming the locked joint 150 are also contemplated herein.

Accordingly, when the stent lattice 100 is deployed or expanded to the open position (open configuration of the stent 100), adjacent hoops 106a-106h interlock with each other at the newly formed joints 150 mateably connecting adjacent hoops 106a - 106h. For example, when the stent lattice 100 is moved to its open configuration, the hoop 106b mateably connects or interlocks with the adjacent hoop 106c and the hoop 106c interlocks with the adjacent hoop 106d, etc. Thus, the points of interlocking or mateable connection are located at the newly formed locked joint 150 between each pair of adjacent hoops 106 as shown. Thus, each locked joint 150 is formed by at least one loop 110 of one hoop 106 (for example 106b, wherein the male end 130 of this loop 110 mateably connects with the female end 140 of another loop 110), i.e. an adjacent loop on an adjacent hoop 106, for example loop 110 on the hoop 106c which is directly opposed from the male end 130 of loop 110 of the hoop 106b. Therefore, the adjacent hoops 106a-106h, are mateably connected to or locked to each other respectively at each locked joint 150 formed in a manner such as described above.

Upon the mateable connection or linking of the male end 130 to the female end 140 (on the loops 110 of adjacent hoops 106), a formed cell 120c is created or formed between adjacent locked joints 150 form by a pair of interlocking, adjacent hoops 106, for example, 106a and 106b, etc. Each formed cell 120c is a fully enclosed area or space defined by the struts 108 loops 110 and locked joints 150 formed by the adjacent hoops 106, i.e. linking of hoop 106a to hoop 106b, linking of hoop 106b to adjacent hoop 106c, etc. Accordingly, the partial cell 120b (Fig. 2) of the stent lattice 100 in its crimped configuration, becomes the formed cell 120c when linked or coupled by the locked joint 150 between adjacent hoops 106 as shown in Fig. 4.

The stent 100 has flexible links 110 that may be on one or more of the following components of the stent lattice: the hoops 106a - 106h, the loops 110, and/or the struts 108. Moreover, the components of the stent lattice, i.e. hoops, loops, struts and flexible links, have drug coatings or drug and polymer coating combinations that are used to deliver drugs, i.e. therapeutic and/or pharmaceutical agents including: antiproliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP)II_{b}IIIₐ inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); antiinflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetominophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF) platelet derived growth factor (PDGF), erythropoetin,; angiotensin receptor blocker; nitric oxide donors; anti-sense oligionucleotides and combinations thereof; cell cycle inhibitors, mTOR inhibitors, and growth factor signal transduction kinase inhibitors. It is important to note that one or more of the lattice components (e.g. hoops, loops, struts and flexible links) are coated with one or more of the drug coatings or drug and polymer coating combinations. Additionally, as mentioned above, the stent 100 is alternatively made of a polymer material itself such as a biodegradable material capable of containing and eluting one or more drugs, in any combination, in accordance with a specific or desired drug release profile.

The method of utilizing the stent 100 includes first identifying a location, for example, a site within the vessel in a patient's body for deployment of the stent 100. Upon identifying the desired deployment location, for example a stenotic lesion or vulnerable plaque site, a delivery device, such as a catheter carrying the stent 100 crimped to a distal end of the catheter such that the stent 100 is in its closed configuration, is inserted within the vessel in the patient's body. The catheter is used to traverse the vessel until reaching the desired location (site) wherein the distal end of the catheter is positioned at the desired location (site), for instance the lesion, within the vessel. At this point, the stent 100 is deployed to its open configuration by expanding the stent 100 such as by inflation if the stent 100 is a balloon expandable stent or by uncovering or release of the stent 100 if the stent 100 is a self-expanding (crush recoverable) type stent. If a cover is utilized to further protect and secure the stent 100 to the catheter distal end when the stent 100 is a self-expanding stent, the cover is removed from the distal end of the catheter prior to expansion of the stent 100, for instance, through use of an expandable member such as an inflatable balloon.

Upon expanding the stent 100 to its open configuration, the expandable member (balloon) is then collapsed, for instance through deflation of the expandable member, whereby the catheter is removed from the deployment site of the vessel and patient's body altogether.

As mentioned previously, the design of the stent 100, i.e. the interlocking of adjacent hoops 106 upon deployment of the stent 100, allows for a wide array of materials, not previously used with stents, to be used with the stent 100. These include materials normally prone to crushing, deformation or recoil upon deployment of the stent. These materials include plastics and polymers to include biodegradable polymers such as drug eluting polymers.

The stent shown in Figs. 1-4 does not form part of the invention as claimed below.

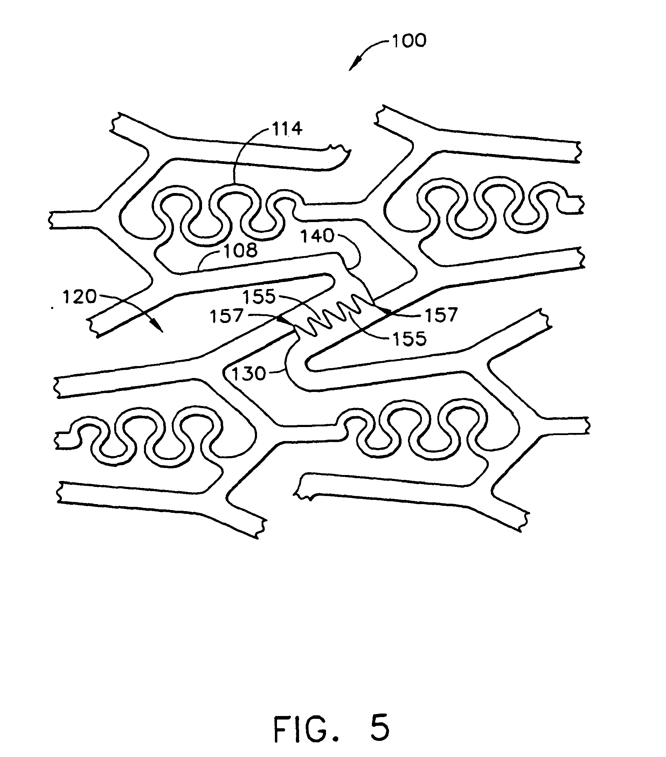

An embodiment for the stent 100 in accordance with the present invention is best depicted in Fig. 5, Figs. 6A - 6E, and Fig. 7. The stent 100 in accordance with this embodiment of the present invention has the same or substantially similar features, elements and their functions as detailed above for the stent of Figs. 1-4 above. Likewise, the same reference numerals are used to designate like or similar features and their function for the stent embodiment of Figs. 5, 6A - 6E and 7 in accordance with the present invention.

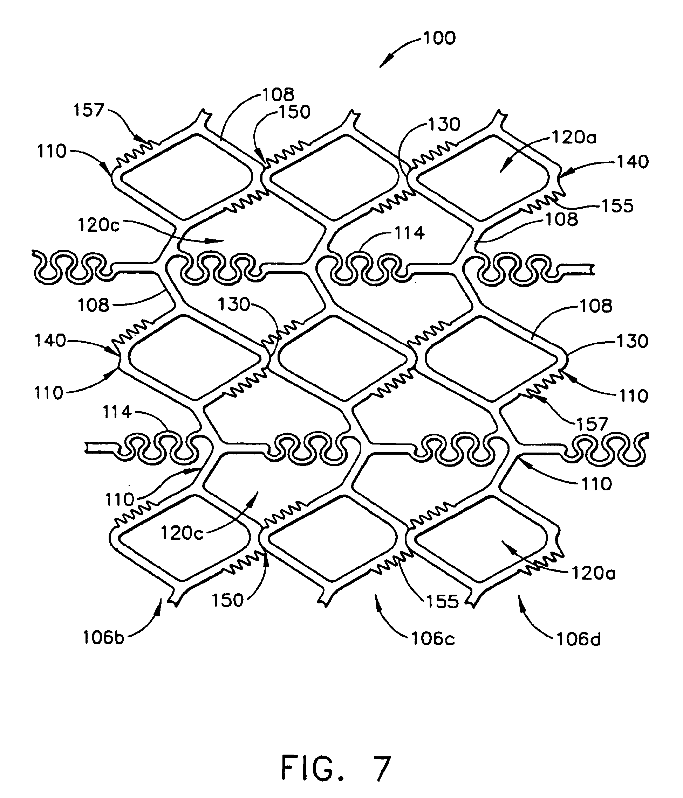

Fig. 5 and Fig. 7 are partial, enlarged views that illustrate the stent 100 having one or more struts 108 on adjacent hoops 106 wherein these struts 108 have a plurality of teeth 155 arranged along the outer edge or outer surface of these struts 108. The teeth 155 of adjacent struts 108 of adjacent hoops 106 as shown are designed such that the teeth 155 of the respective struts 108 are in interlocking engagement (mateably connectable) or mesh with each other at a plurality of locking points 157. The locking points 157 are defined by a tip of one of the teeth 155 received in a tip receiving area or notch on the opposite strut 108 (of an adjacent hoop 106) wherein the area of this strut 108 is shaped to receive the tips of the teeth 155 of the opposite strut 108 of the adjacent hoop 106.

Accordingly, this arrangement as shown in Fig. 5, clearly depicts interlocking adjacent struts 108. All teeth 155 of one strut 108 are moveably received in the tip receiving areas 157, i.e. locking points, when the stent 100 is in the closed configuration. Accordingly, when the stent 100 is in the closed configuration, all teeth 155 of one strut 108 are seated or fit within their locking points 157 of the opposed strut 108 on the adjacent hoop 106.

Although Fig. 5, Fig. 6A - 6E, and Fig. 7 depict the interlocking adjacent struts 108 having a total of five teeth respectively, the adjacent interlocking struts 108 are not limited to any particular number of teeth, but rather comprise one or more teeth respectively as desired. Moreover, the present invention is not limited to the saw tooth or serrated edge embodiment 155 for the interlocking adjacent struts 108, but rather, includes any configuration (for example, sinusoidal, dove tail, tongue and groove, etc.) for the interlocking teeth 155, so long as the interlocking adjacent struts 108 have multiple and discrete locking points 157 that permit the stent 100 to be opened to a plurality of discrete or separate locked positions. Each of these discrete or separate locked positions serve as the open configuration for stent 100 (Figs. 4-7) if desired.

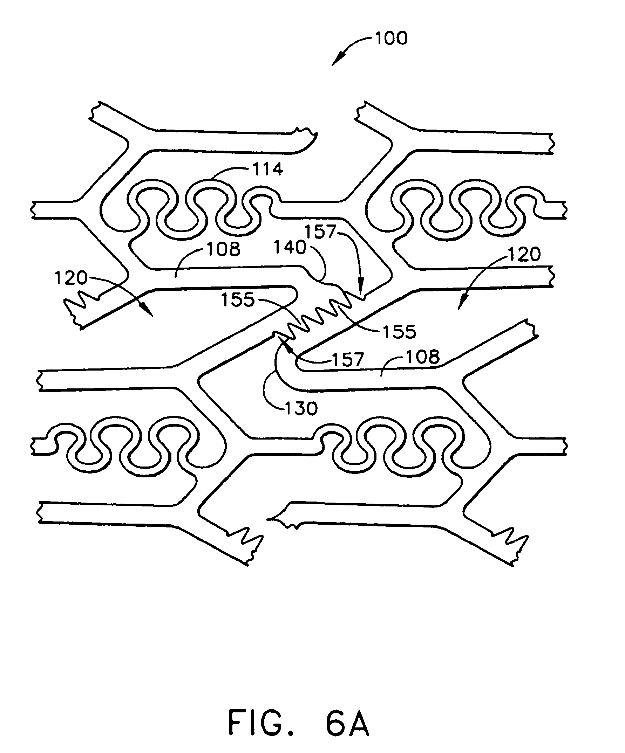

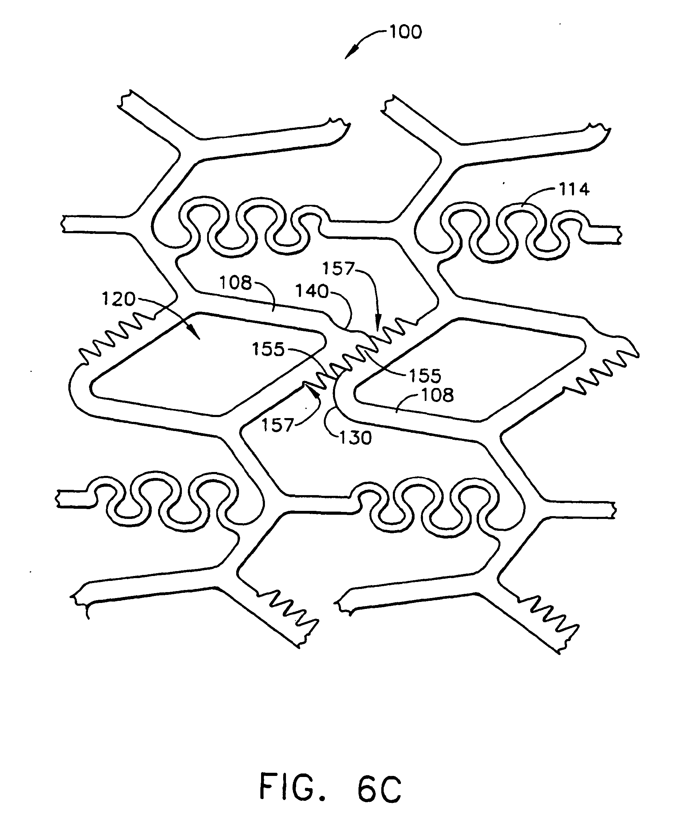

Figs. 6A - 6E depict the stent 100 at various stages of locked movement as the stent 100 is lockably moved from its closed configuration (Fig. 5) to its final open configuration (Fig. 7). As shown in Figs. 6A - 6E, as the stent 100 is expanded from its closed configuration to its open configuration, each notch 157 is exposed as the teeth 155 of the adjacent interlocking struts 108 are interlockingly moved, indexed or ratcheted through the various locking points 157 as shown. It is important to note that stent 100 can be either locked in its closed configuration as shown in Fig. 5 or unlocked in its closed configuration; i.e. no teeth 155 engaged with a respective notch 157 (not shown).

The interlocking adjacent struts 108 each have an interlockable edge, i.e. a serrated edge or teeth 155, in this example, along their common sides that allow for multiple locking interactions between the diamond-shape cells 120 and 120a (Fig. 7) as the diameter of the stent 100 is increased, e.g. through expanding the stent 100 from its closed configuration (Fig. 5) to its final open configuration (Fig. 7). As shown in Figs. 6A - 6E, the teeth or serrated edges 155 engage the opposed struts 108 of adjacent hoops 106 at their common interlockable edges such that the two adjacent cells 120 on the respective adjacent hoops 106 move parallel to one another during expansion of the stent 100.

In accordance with the present invention, the stent embodiment depicted in Fig. 5, Figs. 6A - 6E and Fig. 7 results in a stent having a highly selectable, customizable locking design that permits the stent 100 to be opened to any desired locked diameter, i.e. an open position that is a locked position at various diameter sizes.

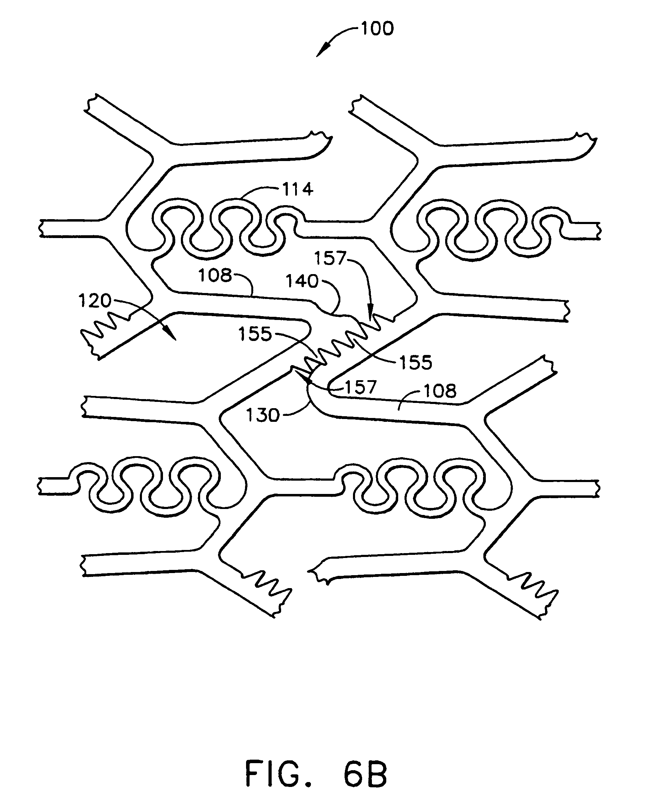

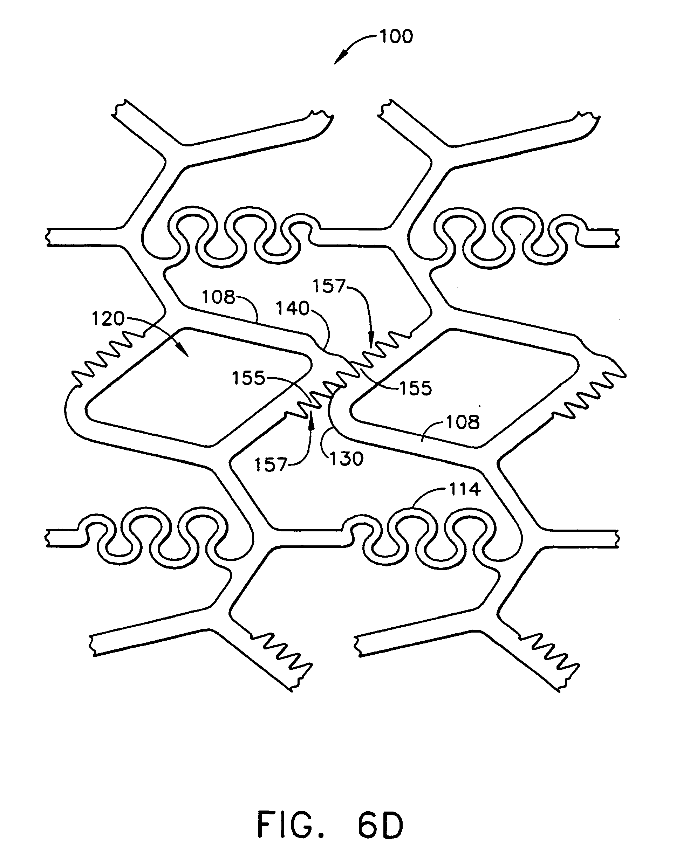

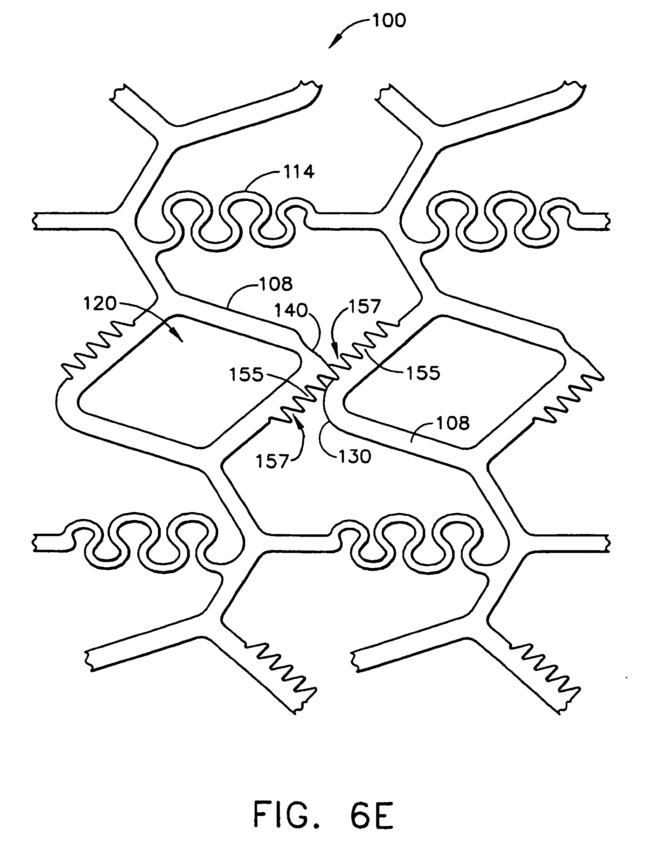

Accordingly, as illustrated, the stent 100 is deployed to a plurality of distinct, variable diameters (increasing size diameters). For example, the stent 100 in accordance with the present invention is lockingly expandable from its closed configuration (Fig. 5) to one of six different and distinct stent diameters (open configuration) as shown in Figs. 6A, 6B, 6D, 6E, and Fig. 7 respectively. As mentioned above, these different and distinct stent diameters increase in size at each different locking point 157 and 150 (Fig. 7) respectively.

Moreover, similar to the stent 100 depicted in Figs. 1-4, the embodiment of the stent 100 depicted in Figs. 5, 6A - 6E and 7, is also made of these previously described material to include alloys such as stainless steel and nickel titanium (NiTi) or polymers such as biodegradable polymers. Additionally, the stent 100 embodiment depicted in Figs. 5, 6A - 6E and Fig. 7, also comprise a drug or therapeutic agent such as those described previously in this disclosure which include rapamycin, paclitaxel or any of the other previously identified therapeutic agents, chemical compounds, biological molecules, nucleic acids such as DNA and RNA, peptides, proteins or combinations thereof.

The stent 100 can be made from biodegradable or bioabsorbable polymer compositions. The type of polymers used can degrade via different mechanisms such as bulk or surface erosion. Bulk erodible polymers include aliphatic polyesters such poly (lactic acid); poly (glycolic acid); poly (caprolactone); poly (p-dioxanone) and poly (trimethylene carbonate); and their copolymers and blends. Other polymers can include amino acid derived polymers; phosphorous containing polymers [e.g., poly (phosphoesters)] and poly (ester amide). Surface erodible polymers include polyanhydrides and polyorthoesters. The stent 100 can be made from combinations of bulk and surface erodible polymers to control the degradation mechanism of the stent. For example, the regions (e.g., interlocks 155 and 157) that are under high stress can be made from a polymer that will retain strength for longer periods of time, as these will degrade earlier than other regions with low stress. The selection of the polymers will determine the absorption of stents 100 that can be very short (few weeks) and long (weeks to months).

The bioabsorbable compositions to prepare the stent 100 will also include drug and radiopaque materials. The amount of drug can range from about 1 to 30% as an example, although the amount of drug loading can comprise any desired percentage. The stent 100 will carry more drug than a polymer-coated stent. The drug will release by diffusion and during degradation of the stent 100. The amount of drug release will be for a longer period of time to treat local and diffuse lesions; and for regional delivery for arterial branches to treat diseases such as vulnerable plaque. Radiopaque additives can include barium sulfate and bismuth subcarbonate and the amount can be from 5 to 30% as an example.

Other radiopaque materials include gold particles and iodine compounds. The particle size of these radiopaque materials can vary from nanometers to microns. The benefits of small particle size is to avoid any reduction in the mechanical properties and to improve the toughness values of the devices. Upon polymer absorption, small particles will also not have any adverse effects on surrounding tissues.

The tubes to prepare bioabsorbable stents 100 can be fabricated either by melt or solvent processing. The preferred method will be solvent processing, especially for the stents that will contain drug. These tubes can be converted to the desired design by excimer laser processing. Other methods to fabricate the stent can be injection molding using supercritical fluids such as carbon dioxide.

The bioabsorbable stents can be delivered by balloon expansion; self-expansion; or a balloon assist self expansion delivery system. The benefit of using the combination system is that the stent does not have to be crimped to lower profiles and upon deployment the stent will self expand to a certain value and can be further expanded to the desired dimension by balloon expansion in accordance with the present invention as best shown in Figs. 4-7.

In accordance with the present invention, the embodiment of the stent 100 depicted in Fig. 5, Figs. 6A - 6E and Fig. 7 also provide for increased radial strength for the stent 100 such that the mechanical locking action of the cells 120 and 120a increase the radial strength of the stent 100 in a manner that exceeds the radial strength associated with the prior art stent designs.

Moreover, since the substantially diamond-shaped cells 120 and 120a of the stent 100 in accordance with the present invention are not connected to one another along the axis of the stent, the length of the stent 100 will not decrease or will only exhibit minimal foreshortening as these cells 120 and 120a contract upon deployment of the stent 100.

Furthermore, the mechanical locking action of the cells 120 and 120a prevent the stent 100 from compressing axially, i.e. compression along the longitudinal axis of the stent 100 thereby resulting in increased column strength for the stent 100 in a manner that exceeds the column strength normally associated with the prior art stent designs.

Furthermore, the interlocking adjacent struts 108, due to their respective serrated edges 155 and locking points 157 assist in locking the stent 100 at its smallest diameter while the stent 100 is crimped onto a delivery device such as a catheter, i.e. while the stent 100 is crimped onto the balloon of the delivery catheter. Accordingly, this mating or interlocking of the interlocking adjacent struts 108 (due to their serrated edges 155) prevents the stent 100 from expanding or deploying prematurely until the moment where sufficient force is applied by the inflation of the balloon in order to overcome the resistance caused by the interlocking of the serrations of teeth 155 of the interlocking adjacent struts 108.

Additionally, in accordance with the present invention, the interlocking adjacent struts 108 can have teeth 155 of any desired shape or configuration and any desired number of serrations along the common side of each diamond-shaped cell 120 and 120a in order to increase or decrease the amount of force that is required to either initiate expansion of the stent 100 or to customize or tailor the radial strength of the stent 100 at each of these distinct, locked positions. The number of serrations can also be modified to either increase or decrease the number of distinct interlocking positions of two adjacent cells 120 and 120a.

While preferred embodiments of the present invention have been shown and described herein, it will be obvious to those skilled in the art that such embodiments are provided by way of example only. Numerous variations, changes, and substitutions will now occur to those skilled in the art without departing from the invention. Accordingly, it is intended that the invention be limited only by the scope of the appended claims.

## Claims

1. A stent (100) comprising:
a lattice of interconnecting elements defining a substantially cylindrical configuration having a first open end and a second open end, the lattice having a closed configuration having a first length and a first diameter and a final open configuration having the first length, but instead having a second diameter, which second diameter is larger than the first diameter;
the lattice comprising a plurality of adjacent hoops (106), and **characterised by** each hoop (106) interlocking with another hoop (106) at a plurality of distinct points (157) during the movement of the lattice from the closed configuration to the final open configuration wherein by interlocking at said distinct points, the lattice has additional, distinct, open configurations, having the first length and a distinct diameter, larger than the first diameter but smaller than the second diameter.

2. The stent (100) according to Claim 1, wherein at least one hoop (106) interlocks with another hoop (106) when the lattice is in the final open configuration.

3. The stent (100) according to any one of the preceding claims, wherein each hoop (106) comprises a plurality of loops.

4. The stent (100) according to Claim 3, wherein each hoop (106) further comprises a plurality of struts (108) connected to the loops.

5. The stent (100) according to Claim 4, wherein the interlocking at the plurality of distinct points (157) is in the form of at least one strut (108) of one hoop (106) interlocking with a strut (108) of an adjacent hoop (106), the at least one strut (108) of one hoop (106) interlocking with the strut (108) of an adjacent hoop (106) defining interlocking adjacent struts (108).

6. The stent (100) according to Claim 5, wherein the interlocking adjacent struts (108) interlock with each other at a plurality of points (157) at at least one of the distinct points (157) during the movement of the lattice from the closed configuration to the final open configuration.

7. The stent (100) according to Claim 5 or Claim 6, wherein the interlocking adjacent struts (108) each comprise a plurality of teeth mateably connectable with each other to a greater or lesser degree as the lattice is moved between the closed configuration and each additional, distinct, open configuration.

8. The stent (100) according to any one of Claims 3 to 7, wherein at least one loop of one hoop (106) comprises a male end and at least one loop of another hoop (106) comprises a female end, wherein the male end is separated from the female end when the lattice is in the closed configuration and wherein the male end is mateably connected to the female end when the lattice is in the final open configuration.

9. The stent (100) according to Claim 8, wherein the male end of at least one loop of one hoop (106) and the female end of at least one loop of another hoop (106) form a locked joint when the lattice is in the final open configuration.

10. The stent (100) according to Claim 8 or Claim 9, wherein the male end has a substantially convex configuration.

11. The stent (100) according to Claim 10, wherein the female end has a substantially concave configuration.

12. The stent (100) according to any one of Claims 3 to 11, wherein the lattice further comprises at least one flexible link connected between adjacent hoops (106).

13. The stent (100) according to Claim 12, wherein the at least one flexible link is connected between the loops of adjacent hoops (106).

14. The stent (100) according to any one of Claims 4 to 13, wherein the plurality of struts (108) and the loops define at least one pre-configured cell.

15. The stent (100) according to Claim 14, wherein the at least one pre-configured cell has a substantially diamond shape.

16. The stent (100) according to any one of Claims 4 to 15, wherein the plurality of struts (108) and the loops define at least one partial cell.

17. The stent (100) according to Claim 16, wherein the partial cell is defined when the lattice is in the closed configuration.

18. The stent (100) according to any one of Claims 4 to 15, wherein the plurality of struts (108) and the loops define at least one formed cell.

19. The stent (100) according to Claim 18, wherein the at least one formed cell is defined when the lattice is in the open configuration.

20. The stent (100) according to any one of the preceding Claims, wherein the lattice further comprises a drug coating.

21. The stent (100) according to any one of the preceding Claims, wherein the lattice further comprises a drug and polymer coating combination.

22. The stent (100) according to Claim 20 or Claim 21, wherein the drug is rapamycin.

23. The stent (100) according to Claim 20 or Claim 21, wherein the drug is paclitaxel.

24. The stent (100) according to any one of the preceding Claims, wherein the stent (100) is made of an alloy.

25. The stent (100) according to Claim 24, wherein the stent (100) is made of stainless steel.

26. The stent (100) according to any one of the preceding Claims, wherein the stent (100) is crush recoverable.

27. The stent (100) according to Claim 24, wherein the stent (100) is made of a super elastic alloy.

28. The stent (100) according to Claim 26 or 27, wherein the stent (100) is made of nickel titanium (NiTi).

29. The stent (100) according to any one of Claims 1 to 23, wherein the stent (100) is made of a polymer.

30. The stent (100) according to Claim 29, wherein the stent (100) is made of a biodegradable polymer.

31. The stent (100) according to Claim 30, further comprising a drug.

32. The stent (100) according to Claim 31, wherein the drug is rapamycin.

33. The stent (100) according to Claim 31, wherein the drug is paclitaxel.

34. The stent (100) according to any one of Claims 30 to 33, wherein the polymer is a bulk erodible polymer.

35. The stent (100) according to any one of Claims 30 to 33, wherein the polymer is a surface erodible polymer.

36. The stent (100) according to any one of the preceding Claims, further comprising a radiopaque material.

37. The stent (100) according to any one of the preceding Claims, wherein at least one hoop (106) interlocks with another hoop (106) in the closed configuration.

## Patentansprüche

1. Ein Stent (100), umfassend:
- ein Netz aus Verbindungselementen, welche eine im Wesentlichen zylindrische Konfiguration mit einem ersten offenen Ende und einem zweiten offenen Ende bilden, wobei das Netz eine geschlossene Konfiguration mit einer ersten Länge und einem ersten Durchmesser und eine offene Endkonfiguration mit einer ersten Länge aber mit einem zweiten Durchmesser aufweist, welcher größer ist, als der erste Durchmesser;
- wobei das Netz mehrere benachbarte Schleifen (106) umfaßt und **dadurch gekennzeichnet ist, daß** jede Schleife (106) und eine andere Schleife (106) während der Bewegung des Netzes von der geschlossenen Konfiguration in die offene Endkonfiguration an mehreren eindeutigen Punkten (157) ineinandergreifen, wobei mittels des Ineinandergreifens der eindeutigen Punkte das Netz zusätzliche, eindeutige, offene Konfigurationen mit der ersten Länge und einem eindeutigen Durchmesser aufweist, welcher größer ist, als der erste Durchmesser, aber kleiner, als der zweite Durchmesser.

2. Der Stent (100) nach Anspruch 1, **dadurch gekennzeichnet, daß** zumindest eine Schleife (106) und eine andere Schleife (106) ineinandergreifen, wenn das Netz in der offenen Endkonfiguration ist.

3. Der Stent (100) nach einem der vorangehenden Ansprüche, **dadurch gekennzeichnet, daß** jede Schleife (106) mehrere Schlingen aufweist.

4. Der Stent (100) nach Anspruch 3, **dadurch gekennzeichnet, daß** jede Schleife (106) ferner mehrere mit den Schlingen verbundene Streben (108) aufweist.

5. Der Stent (100) nach Anspruch 4, **dadurch gekennzeichnet, daß** das Ineinandergreifen an den mehreren eindeutigen Punkten (157) in der Form vorliegt, daß zumindest eine Strebe (108) einer Schleife (106) und eine Strebe (108) einer benachbarten Schleife (106) ineinandergreifen, wobei die zumindest eine Strebe (108) einer Schleife (106) und die Strebe (108) der benachbarten Schleife (106) ineinandergreifende benachbarte Streben (108) bilden.

6. Der Stent (100) nach Anspruch 5, **dadurch gekennzeichnet, daß** die ineinandergreifenden benachbarten Streben (108) an mehreren Punkten (157) an zumindest den eindeutigen Punkten (157) während der Bewegung des Netzes von der geschlossenen Konfiguration in die offene Endkonfiguration ineinandergreifen.

7. Der Stent (100) nach Anspruch 5 oder Anspruch 6, **dadurch gekennzeichnet, daß** die ineinandergreifenden benachbarten Streben (108) jeweils mehrere Zähne aufweisen, welche zu einem höheren oder geringeren Maße miteinander zusammenfügbar verbindbar sind, wenn das Netz zwischen der geschlossenen Konfiguration und jeder zusätzlichen, eindeutigen, offenen Endkonfiguration bewegt wird.

8. Der Stent (100) nach einem der Ansprüche 3 bis 7, **dadurch gekennzeichnet, daß** zumindest eine Schlinge einer Schleife (106) ein männliches Ende und zumindest eine Schlinge einer anderen Schleife (106) ein weibliches Ende aufweist, wobei das männliche Ende von dem weiblichen Ende getrennt ist, wenn das Netz in der geschlossenen Konfiguration ist, und wobei das männliche Ende mit dem weiblichen Ende zusammenfügbar verbindbar ist, wenn das Netz in der offenen Endkonfiguration ist.

9. Der Stent (100) nach Anspruch 8, **dadurch gekennzeichnet, daß** das männliche Ende zumindest einer Schlinge einer Schleife (106) und das weibliche Ende zumindest einer Schlinge einer anderen Schleife (106) ein gesperrtes Gelenk bilden, wenn das Netz in der offenen Endkonfiguration ist.

10. Der Stent (100) nach Anspruch 8 oder Anspruch 9, **dadurch gekennzeichnet, daß** das männliche Ende eine im Wesentlichen konvexe Konfiguration aufweist.

11. Der Stent (100) nach Anspruch 10, **dadurch gekennzeichnet, daß** das weibliche Ende eine im Wesentlichen konkave Konfiguration aufweist.

12. Der Stent (100) nach einem der Ansprüche 3 bis 11, **dadurch gekennzeichnet, daß** das Netz ferner zumindest eine flexible Verbindung aufweist, welche zwischen benachbarten Schleifen (106) verbunden ist.

13. Der Stent (100) nach Anspruch 12, **dadurch gekennzeichnet, daß** die zumindest eine flexible Verbindung zwischen den Schlingen benachbarter Schleifen (106) verbunden ist.

14. Der Stent (100) nach einem der Ansprüche 4 bis 13, **dadurch gekennzeichnet, daß** die mehreren Streben (108) und die Schlingen zumindest eine vor-konfigurierte Zelle bilden.

15. Der Stent (100) nach Anspruch 14, **dadurch gekennzeichnet, daß** die zumindest eine vor-konfigurierte Zelle im Wesentlichen diamantförmig ist.

16. Der Stent (100) nach einem der Ansprüche 4 bis 15, **dadurch gekennzeichnet, daß** die mehreren Streben (108) und die Schlingen zumindest eine partielle Zelle milden.

17. Der Stent (100) nach Anspruch 16, **dadurch gekennzeichnet, daß** die partielle Zelle gebildet ist, wenn das Netz in der geschlossenen Konfiguration ist.

18. Der Stent (100) nach einem der Ansprüche 4 bis 15, **dadurch gekennzeichnet, daß** die mehreren Streben (108) und die Schlingen zumindest eine geformte Zelle bilden.

19. Der Stent (100) nach Anspruch 18, **dadurch gekennzeichnet, daß** die zumindest eine geformte Zelle gebildet ist, wenn das Netz in der offenen Konfiguration ist.

20. Der Stent (100) nach einem der vorangehenden Ansprüche, **dadurch gekennzeichnet, daß** das Netz ferner eine Arzneimittelbeschichtung aufweist.

21. Der Stent (100) nach einem der vorangehenden Ansprüche, **dadurch gekennzeichnet, daß** das Netz ferner eine Kombination einer Arzneimittel- und Polymerbeschichtung aufweist.

22. Der Stent (100) nach Anspruch 20 oder Anspruch 21, **dadurch gekennzeichnet, daß** das Arzneimittel Rapamycin ist.

23. Der Stent (100) nach Anspruch 20 oder Anspruch 21, **dadurch gekennzeichnet, daß** das Arzneimittel Paclitaxel ist.

24. Der Stent (100) nach einem der vorangehenden Ansprüche, **dadurch gekennzeichnet, daß** der Stent (100) aus einer Legierung gebildet ist.

25. Der Stent (100) nach Anspruch 24, **dadurch gekennzeichnet, daß** der Stent (100) aus Edelstahl gebildet ist.

26. Der Stent (100) nach einem der vorangehenden Ansprüche, **dadurch gekennzeichnet, daß** der Stent (100) stoßelastisch ist.

27. Der Stent (100) nach Anspruch 24, **dadurch gekennzeichnet, daß** der Stent (100) aus einer superelastischen Legierung gebildet ist.

28. Der Stent (100) nach Anspruch oder 27, **dadurch gekennzeichnet, daß** der Stent (100) aus Nickel-Titan (NiTi) gebildet ist.

29. Der Stent (100) nach einem der Ansprüche 1 bis 21, **dadurch gekennzeichnet, daß** der Stent (100) aus einem Polymer gebildet ist.

30. Der Stent (100) nach Anspruch 29, **dadurch gekennzeichnet, daß** der Stent (100) aus einem biologisch abbaubaren Polymer gebildet ist.

31. Der Stent (100) nach Anspruch 30, ferner **gekennzeichnet durch** ein Arzneimittel.

32. Der Stent (100) nach Anspruch 31, **dadurch gekennzeichnet, daß** das Arzneimittel Papamycin ist.

33. Der Stent (100) nach Anspruch 31, **dadurch gekennzeichnet, daß** das Arzneimittel Paclitaxel ist.

34. Der Stent (100) nach einem der Ansprüche 30 bis 33, **dadurch gekennzeichnet, daß** das Polymer ein volumenerodierbares Polymer ist.

35. Der Stent (100) nach einem der Anspüche 30 bis 33, **dadurch gekennzeichnet, daß** das Polymer ein oberflächenerodierbares Polymer ist.

36. Der Stent (100) nach einem der vorangehenden Ansprüche, ferner **gekennzeichnet durch** ein röntgendichtes Material.

37. Der Stent (100) nach einem der vorangehenden Ansprüche, **dadurch gekennzeichnet, daß** zumindest eine Schleife (106) und eine andere Schleife (106) in der geschlossenen Konfiguration ineinandergreifen.

## Revendications

1. Endoprothèse (100) comprenant :
un maillage d'éléments interconnectés définissant une configuration sensiblement cylindrique comportant une première extrémité ouverte et une seconde extrémité ouverte, le maillage ayant une configuration fermée ayant une première longueur et un premier diamètre, et une configuration ouverte finale ayant la première longueur mais un second diamètre, lequel second diamètre est supérieur au premier diamètre ;
le maillage comprenant une pluralité de mailles adjacentes (106),
et **caractérisée en ce que** chaque maille (106) est couplée à une autre maille (106) en une pluralité de points distincts (157) durant le passage du maillage de la configuration fermée à la configuration ouverte finale,
dans laquelle, en couplant les mailles en lesdits points distincts, le maillage prend des configurations ouvertes supplémentaires, distinctes, ayant la première longueur et un diamètre distinct, supérieur au premier diamètre mais inférieur au second diamètre.

2. Endoprothèse (100) selon la revendication 1, dans laquelle au moins une maille (106) est couplée à une autre maille (106) quand le maillage est dans la configuration ouverte finale.

3. Endoprothèse (100) selon l'une quelconque des revendications précédentes, dans laquelle chaque maille (106) comprend une pluralité de boucles.

4. Endoprothèse (100) selon la revendication 3, dans laquelle chaque maille (106) comprend en outre une pluralité de fourches (108) reliées aux boucles.

5. Endoprothèse (100) selon la revendication 4, dans laquelle le couplage en la pluralité de points distincts (157) a la forme d'au moins une fourche (108) couplant une maille (106) à une fourche (108) d'une maille adjacente (106), la au moins une fourche (108) couplant une maille (106) à la fourche (108) d'une maille adjacente (106) définissant des fourches de couplage adjacentes (108).

6. Endoprothèse (100) selon la revendication 5, dans laquelle les fourches de couplage adjacentes (108) sont couplées les unes aux autres en une pluralité de points (157), et au moins en l'un des points distincts (157), durant le passage du maillage de la configuration fermée à la configuration ouverte finale.

7. Endoprothèse (100) selon la revendication 5 ou la revendication 6, dans laquelle les fourches de couplage adjacentes (108) comprennent chacune une pluralité de dents accouplées les unes aux autres à un degré plus ou moins grand, à mesure que le maillage passe de la configuration fermée à chaque configuration ouverte distincte supplémentaire.

8. Endoprothèse (100) selon l'une quelconque des revendications 3 à 7,
■ dans laquelle au moins une boucle d'une maille (106) comprend une extrémité mâle et au moins une boucle d'une autre maille (106) comprend une extrémité femelle,
■ dans laquelle l'extrémité mâle est séparée de l'extrémité femelle quand le maillage est dans la configuration fermée,
■ et dans laquelle l'extrémité mâle est accouplée à l'extrémité femelle quand le maillage est dans la configuration ouverte finale.

9. Endoprothèse (100) selon la revendication 8, dans laquelle l'extrémité mâle d'au moins une boucle d'une maille (106) et l'extrémité femelle d'au moins une boucle d'une autre maille (106) forment une jonction fermée quand le maillage est dans la configuration ouverte finale.

10. Endoprothèse (100) selon la revendication 8 ou la revendication 9, dans laquelle l'extrémité mâle a une configuration sensiblement convexe.

11. Endoprothèse (100) selon la revendication 10, dans laquelle l'extrémité femelle a une configuration sensiblement concave.

12. Endoprothèse (100) selon l'une quelconque des revendications 3 à 11, dans laquelle le maillage comprend en outre au moins un maillon souple reliant des mailles adjacentes (106).

13. Endoprothèse (100) selon la revendication 12, dans laquelle le au moins un maillon souple relie les boucles des mailles adjacentes (106).

14. Endoprothèse (100) selon l'une quelconque des revendications 4 à 13, dans laquelle la pluralité de fourches (108) et les boucles définissent au moins une cellule préconfigurée.

15. Endoprothèse (100) selon la revendication 14, dans laquelle la au moins une cellule préconfigurée a sensiblement la forme d'un diamant.

16. Endoprothèse (100) selon l'une quelconque des revendications 4 à 15, dans laquelle la pluralité de fourches (108) et les boucles définissent au moins une cellule partielle.

17. Endoprothèse (100) selon la revendication 16, dans laquelle la cellule partielle est définie quand le maillage est dans la configuration fermée.

18. Endoprothèse (100) selon l'une quelconque des revendications 4 à 15, dans laquelle la pluralité de fourches (108) et les boucles définissent au moins une cellule formée.

19. Endoprothèse (100) selon la revendication 18, dans laquelle la au moins une cellule formée est définie quand le maillage est dans la configuration ouverte.

20. Endoprothèse (100) selon l'une quelconque des revendications précédentes, dans laquelle le maillage comprend en outre un enduit médicamenteux.

21. Endoprothèse (100) selon l'une quelconque des revendications précédentes, dans laquelle le maillage comprend en outre une combinaison d'enduits médicamenteux et polymères.

22. Endoprothèse (100) selon la revendication 20 ou la revendication 21, dans laquelle le médicament est de la rapamycine.

23. Endoprothèse (100) selon la revendication 20
ou la revendication 21, dans laquelle le médicament est du paclitaxel.

24. Endoprothèse (100) selon l'une quelconque des revendications précédentes, dans laquelle l'endoprothèse (100) est composée d'un alliage.

25. Endoprothèse (100) selon la revendication 24, dans laquelle l'endoprothèse (100) est composée d'acier inoxydable.

26. Endoprothèse (100) selon l'une quelconque des revendications précédentes, dans laquelle l'endoprothèse (100) résiste à la compression.

27. Endoprothèse (100) selon la revendication 24, dans laquelle l'endoprothèse (100) est composée d'un alliage super élastique.

28. Endoprothèse (100) selon la revendication 26
ou 27, dans laquelle l'endoprothèse (100) est composée de nickel et de titane (NiTi).

29. Endoprothèse (100) selon l'une quelconque des revendications 1 à 23, dans laquelle l'endoprothèse (100) est composée d'un polymère.

30. Endoprothèse (100) selon la revendication 29, dans laquelle l'endoprothèse (100) est composée d'un polymère biodégradable.

31. Endoprothèse (100) selon la revendication 30, comprenant également un médicament.

32. Endoprothèse (100) selon la revendication 31,
dans laquelle le médicament est de la rapamycine.

33. Endoprothèse (100) selon la revendication 31,
dans laquelle le médicament est du paclitaxel.

34. Endoprothèse (100) selon l'une quelconque des revendications 30 à 33, dans laquelle le polymère est un polymère érodable en bloc.

35. Endoprothèse (100) selon l'une quelconque des revendications 30 à 33, dans laquelle le polymère est un polymère érodable en surface.

36. Endoprothèse (100) selon l'une quelconque des revendications précédentes, comprenant en outre un matériau radio-opaque.

37. Endoprothèse (100) selon l'une quelconque des revendications précédentes, dans laquelle au moins une maille (106) est couplée à une autre maille (106) dans la configuration fermée.
